**Europäisches Patentamt**

**European Patent Office**

**Office européen des brevets**

(11) Veröffentlichungsnummer : **0 154 223**
**B1**

(12) EUROPÄISCHE PATENTSCHRIFT

(45) Veröffentlichungstag der Patentschrift :
**19.10.88**

(51) Int. Cl.⁴ : **C 07 C 69/86, C 07 C 67/08, C 07 C 65/24, C 07 C 51/347**

(21) Anmeldenummer : **85101598.2**

(22) Anmeldetag : **14.02.85**

(54) Verfahren zur Herstellung von 6-Acetoxy-2-naphthoesäure und von reiner 6-Hydroxy-2-naphthoesäure.

(30) Priorität : 28.02.84 DE 3407100

(43) Veröffentlichungstag der Anmeldung :
**11.09.85 Patentblatt 85/37**

(45) Bekanntmachung des Hinweises auf die Patenterteilung : **19.10.88 Patentblatt 88/42**

(84) Benannte Vertragsstaaten :
**CH DE FR IT LI**

(56) Entgegenhaltungen :
US-A- 4 145 443
US-A- 4 345 095

(73) Patentinhaber : HOECHST AKTIENGESELLSCHAFT
Postfach 80 03 20
D-6230 Frankfurt am Main 80 (DE)

(72) Erfinder : Neeb, Rudolf, Dr.
Am Klingenrain 23
D-6050 Offenbach/Main (DE)
Erfinder : Tronich, Wolfgang, Dr.
Adolf-Guckes-Weg 5
D-6239 Eppstein/Taunus (DE)
Erfinder : Volk, Heinrich, Dr.
Am Weissen Stein 1
D-6368 Bad Vilbel (DE)

EP 0 154 223 B1

## Beschreibung

Die Erfindung liegt auf dem technischen Gebiet der Herstellung von Zwischenprodukten, die beispielsweise zu Kunststoffen weiterverarbeitet werden können. Sie betrifft ein Verfahren zur Herstellung von 6-Acetoxy-2-naphthoesäure und insbesondere ein Verfahren zur Abtrennung der 6-Hydroxy-2-naphthoesäure von den isomeren Hydroxy-naphthoesäuren durch selektive Überführung der 6-Hydroxy-2-naphthoesäure in die entsprechende 6-Acetoxy-2-naphthoesäure mit gegebenenfalls anschließender Hydrolyse.

6-Acetoxy-2-naphthoesäure hat seit einigen Jahren zunehmende Bedeutung für die Herstellung synthetischer Fasern und Kunststoffe mit interessanten technischen Eigenschaften erlangt (vgl. bspw. US-PS 4 161 470, dort insb. Sp. 7, Z. 23-39 und 51-67, und Bsp. I, und US-PS 4 219 461). Über die Synthese der 6-Acetoxy-2-naphthoesäure liegen in der Literatur allerdings nur sehr allgemeine Angaben vor. So ist zum Beispiel aus J. Chem. Soc. (London) 123, 1653 (1923), lediglich zu entnehmen, daß 6-Acetoxy-2-naphthoesäure aus 6-Hydroxy-2-naphthoesäure « mittels Essigsäureanhydrid » hergestellt wird. Diese Verfahrensmaßnahme muß offensichtlich so verstanden werden, daß 6-Hydroxy-2-naphthoesäure mit Essigsäureanhydrid längere Zeit zum Sieden erhitzt wird ; denn diese Verfahrensweise der Acetylierung wurde bereits bei der Herstellung von 2-Acetoxynaphthalin aus 2-Naphthol (s. Berichte der dt. Chem. Ges. 14 (1881), 1602, Anm. 1) und bei der Herstellung der 3-Acetoxy-2-naphthoesäure aus 3-Hydroxy-2-naphthoesäure (s. Ber. loc. cit. 27, 2624 (1894) ; später auch in Ber. 58, 2116 (1925)) praktiziert.

Die Lehre des Standes der Technik ist also dahingehend, daß 6-Acetoxy-2-naphthoesäure durch Acetylierung von 6-Hydroxy-2-naphthoesäure mittels Essigsäureanhydrid unter Ausschluß von Wasser im sauren Bereich bei Temperaturen von oberhalb 100 °C, im allgemeinen beim Siedepunkt des Acetylierungsgemisches, synthetisiert werden kann.

Im Gegensatz zur Herstellung der 6-Acetoxy-2-naphthoesäure ist jedoch die Herstellung deren Ausgangsproduktes, nämlich der 6-Hydroxy-2-naphthoesäure, die durch Kolbe-Schmitt-Reaktion von Kalium-β-naphtholat und Kohlendioxid erfolgt, sehr ausführlich beschrieben (s. US-Patentschriften Nrs. 1 593 816 und 4 287 357). Bei der Herstellung von 6-Hydroxy-2-naphthoesäure durch die Kolbe-Schmitt-Reaktion ist allerdings die Bildung mehr oder weniger großer Anteile an isomeren Hydroxynaphthalin-monocarbonsäuren und Hydroxynaphthalin-dicarbonsäuren, insbesondere beispielsweise die Bildung von 3-Hydroxy-2-naphthoesäure, unvermeidlich. Diese Anteile an unerwünschten isomeren Hydroxy-naphthoesäuren lassen sich zwar durch geeignete Reaktionsführung in gewissem Umfange zurückdrängen, jedoch ist die erwünschte 6-Hydroxy-2-naphthoesäure nur dadurch in reiner Form erhältlich, daß man, zunächst dem Beispiel der US-PS 1 593 816 oder dem Beispiel 1 der US-PS 4 287 357 folgend, die unterschiedlichen Löslichkeitseigenschaften der isomeren Verbindungen ausnutzt und die Abtrennung der 6-Hydroxy-2-naphthoesäure entweder aus heißem Wasser oder in verdünnter Mineralsäure bei einem bestimmten pH-Wert ausführt.

Gemäß dem obengenannten Stand der Technik ergibt sich somit die Synthese der 6-Acetoxy-2-naphthoesäure über drei getrennte Verfahrensstufen bzw. -schritte, nämlich zunächst eine aufwendige reinigende Abtrennung der durch Kolbe-Schmitt-Reaktion gebildeten 6-Hydroxy-2-naphthoesäure von deren isomeren Hydroxynaphthoesäuren, Trocknung des abgetrennten Produktes und sodann Überführung in die gewünschte 6-Acetoxy-2-naphthoesäure durch Erhitzen mit überschüssigem Essigsäureanhydrid. Die Endausbeute über diese gesamten Schritte ist naturgemäß nicht zufriedenstellend.

Es bestand daher ein Bedürfnis nach einem Verfahren, das in einfacher Weise mit optimaler Ausbeute die Herstellung von isomerenreiner 6-Acetoxy-2-naphthoesäure gestattet.

Es wurde nun gefunden, daß man 6-Hydroxy-2-naphthoesäure in einfacher Weise, — und zudem selektiv aus isomeren Beimengungen, — in die 6-Acetoxy-2-naphthoesäure überführen kann, wenn man die wäßrig-alkalische Lösung eines Alkalisalzes, vorzugsweise Dialkalisalzes, der 6-Hydroxy-2-naphthoesäure, die gegebenenfalls noch Beimengungen an isomeren Hydroxynaphthoesäuren enthält, so beispielsweise die wäßrig alkalischen Lösungen eines Carboxylierungsproduktes von Kalium-β-naphtholat, mit Essigsäureanhydrid umsetzt.

Die erfindungsgemäße Verfahrensweise, 6-Hydroxy-2-naphthoesäure nunmehr in wäßrig-alkalischer Lösung mit Essigsäureanhydrid zu acetylieren, somit auch auf Reaktionstemperaturen von oberhalb 100 °C verzichten zu können, hat insbesondere den großen Vorteil, daß die gleichzeitig in der wäßrigen Lösung anwesenden isomeren Hydroxynaphthoesäuren, insbesondere die 3-Hydroxy-2-naphthoesäure, in überraschender Weise nicht mit Essigsäureanhydrid reagieren, so daß die 6-Hydroxy-2-naphthoesäure selektiv leicht acetyliert und von den übrigen Hydroxynaphthoesäuren abgetrennt werden kann. Die vorliegende Erfindung betrifft somit auch die Abtrennung der 6-Hydroxy-2-naphthoesäure von deren isomeren Verbindungen durch selektive Acetylierung. Die 6-Acetoxy-Verbindung kann nach ihrer Abtrennung gewünschten- oder erforderlichenfalls zur 6-Hydroxy-Verbindung hydrolysiert werden.

Die Alkalisalze der 6-Hydroxy-2-naphthoesäure, die in wäßriger Lösung als Ausgangsverbindungen dienen, sind bevorzugt die Natrium- und die Kaliumsalze.

Die Acetylierungsreaktion wird im stark alkalischen Bereich durchgeführt, vorzugsweise bei einem pH-Wert von 10 oder größer als 10, wie bei einem pH-Wert von 10 bis 13, insbesondere bei einem pH-Wert zwischen 11,5 und 12,5. Die Reaktionstemperatur der Acetylierung kann unterhalb 60 °C liegen, bevorzugt arbeitet man bei einer Temperatur von unterhalb 50 °C, da bei deutlich höheren Temperaturen die Verseifung (Hydrolyse) des Essigsäureanhydrids zu Essigsäure nachteilig in den Vordergrund tritt. Diesbezüglich sollte deshalb zur Vermeidung eines zu hohen Überschusses an Essigsäureanhydrid die Reaktionsführung der Acetylierung bei einer Temperatur zwischen 10 und 40 °C, bevorzugt zwischen 15 und 30 °C, insbesondere zwischen 20 und 25 °C, somit im Bereich der Raumtemperatur, gewählt werden.

Die erfindungsgemäße Acetylierungsreaktion läßt sich im Prinzip mit äquimolaren Mengen an Essigsäureanhydrid und 6-Hydroxy-2-naphthoesäure in zufriedenstellenden Ausbeuten ausführen. Bevorzugt wird jedoch Essigsäureanhydrid im Überschuß verwendet, so in einem bis zu 3,5-fach molaren Überschuß. Denn der Einsatz von überschüssigem Essigsäureanhydrid bringt Vorteile für die nachfolgenden Aufarbeitungsschritte. So wird in der Regel die Essigsäureanhydridmenge so gewählt, daß die durch die Acetylierung einerseits und die durch die Verseifung des überschüssigen Essigsäureanhydrids andererseits freiwerdende Menge an Essigsäure ausreicht, um die Reaktionsmischung auf einen pH-Wert zwischen etwa 6,0 und 4,0, insbesondere zwischen 5,5 und 4,5 zu bringen, ohne daß nachträglich — in einem weiteren Schritt — zusätzlich Säure zugegeben werden muß. Selbstverständlich kann der pH-Wert nach der Acetylierungsreaktion auch mit einer anderen Säure, wie einer organischen oder anorganischen, hierfür üblichen Säure, vorgenommen werden.

In der Regel verwendet man das Essigsäureanhydrid in einer Menge von 1,2 bis 3,0 Mol, bevorzugt 1,5 bis 2,5 Mol, pro Mol 6-Hydroxy-2-naphthoesäure.

Die nach dem erfindungsgemäßen Verfahren erhältliche 6-Acetoxy-2-naphthoesäure wird aus der Reaktionsmischung bei dem oben angegebenen pH-Wert zwischen etwa 6,0 und 4,0 ausgefällt und beispielsweise durch Filtration isoliert. Stellt man das anfallende Filtrat durch Zusatz einer Säure, beispielsweise verdünnter Schwefelsäure, auf einen pH-Wert zwischen 2 und 1, so lassen sich die in Lösung gebliebenen, unter den erfindungsgemäßen Reaktionsbedingungen nicht acetylierten Naphthoesäuren, wie insbesondere die 3-Hydroxy-2-naphthoesäure, ebenfalls ausfällen und isolieren.

Eine einfache Möglichkeit zur Herstellung der wäßrigen Ausgangslösung der Alkalisalze (Dialkalisalze) der 6-Hydroxy-2-naphthoesäure besteht zum Beispiel darin, daß man die 6-Hydroxy-2-naphthoesäure entweder in reiner Form oder aber im Gemisch mit isomeren Beimengungen, wie beispielsweise mit der 3-Hydroxy-2-naphthoesäure (solche Mischungen fallen beispielsweise bei der Aufarbeitung der wäßrigen Mutterlauge gemäß Beispiel 1 der US-PS 4 287 357 an), in wäßrigem Alkalihydroxid, das bevorzugt 2 Mol Alkalihydroxid pro Mol Hydroxynaphthoesäure enthält, löst. Die Konzentration der Alkalisalze (Dialkalisalze) in dieser wäßrig-alkalischen Lösung richtet sich in der Regel nach deren Löslichkeit bei der jeweils benutzten Temperatur; so kann die 6-Hydroxy-2-naphthoesäure in der Regel im Bereich zwischen etwa 20 und 40 °C in bis zu 20 gew.-%iger wäßriger Lösung, bevorzugt in 3- bis 10 gew.-%iger Lösung, in den Acetylierungsansatz eingesetzt werden.

Eine besonders bevorzugte Ausführungsform des erfindungsgemäßen Verfahrens ist somit die selektive Acetylierung der 6-Hydroxy-2-naphthoesäure aus wäßrig-alkalischen Lösungen von Hydroxynaphthoesäuren, — wie von wäßrig-alkalischen Lösungen aus Carboxylierungsansätzen von Kalium-β-naphtholat oder Mischungen von Kalium- und Natrium-β-naphtholat, die nach literaturbekannten Verfahren unter vorheriger Abtrennung harziger Anteile und nicht umgesetzten β-Naphthols aufgearbeitet wurden und isomere Hydroxynaphthoesäuren als Nebenprodukte, insbesondere 3-Hydroxy-2-naphthoesäure als Nebenprodukt, enthalten.

Erfolgt die erfindungsgemäße Acetylierung in Mischung mit anderen, isomeren Hydroxy-2-naphthoesäuren, so wird Essigsäureanhydrid in der Regel in einer Menge in die Reaktion eingesetzt, die sich entsprechend den genannten Werten auf die Gesamtmenge der isomeren Hydroxy-2-naphthoesäuren berechnet, so in der Regel in der äquimolaren bis 3,5-fach molaren Menge, bevorzugt 1,2- bis 3,0-fach und insbesondere bevorzugt 1,5- bis 2,5-fach molaren Menge, bezogen auf die Gesamtmenge der isomeren Hydroxy-2-naphthoesäuren.

Wie bereits erwähnt, kann die erhaltene 6-Acetoxy-2-naphthoesäure anschließend zur 6-Hydroxy-2-naphthoesäure hydrolysiert werden; hierbei erhält man die 6-Hydroxy-2-naphthoesäure als sehr reines Produkt. Es gelingt auf diese Weise, verunreinigte und/oder mit Isomeren vermischte 6-Hydroxy-2-naphthoesäure auf verhältnismäßig einfachem Wege über die Acetylierung und anschließende Hydrolyse in guter Ausbeute zu reinigen. Dieser Weg hat deutliche Vorteile gegenüber der Reinigung durch Umkristallisation, wie diese beispielsweise gemäß der US-Patentschrift Nr. 1 593 816 durchgeführt wird.

Die vorliegende Erfindung betrifft somit auch ein Verfahren zur Herstellung von reiner 6-Hydroxy-2-naphthoesäure bzw. zur Reinigung von durch insbesondere isomere Hydroxynaphthoesäuren verunreinigter 6-Hydroxy-2-naphthoesäure, das dadurch gekennzeichnet ist, daß man die verunreinigte 6-Hydroxy-2-naphthoesäure zunächst gemäß den obigen erfindungsgemäßen Angaben acetyliert und die isolierte 6-Acetoxy-2-naphthoesäure anschließend hydrolysiert.

Die Hydrolyse der 6-Acetoxy-2-naphthoesäure zur 6-Hydroxy-2-naphthoesäure kann in an und für sich bekannter Weise erfolgen, so in wäßrig-alkalischem Medium oder in wäßrig-saurem Medium. Hierbei kann, insbesondere bei der Hydrolyse in wäßrig-saurem Medium, ein in Wasser lösliches oder mischbares, in diesem Reaktionsansatz inertes Lösemittel zugesetzt werden, um die Löslichkeit der 6-Acetoxy-2-naphthoesäure in dem Reaktionsmedium zu verbessern. Solche Lösemittel sind insbesondere geradkettige oder verzweigte Alkanole von 1 bis 5 C-Atomen, wie beispielsweise Ethanol.

Als wäßrig-alkalisches Hydrolysemedium kann man beispielsweise eine wäßrige Ammoniumhydroxidlösung oder eine wäßrige Alkalihydroxidlösung, wie Natronlauge, verwenden. Hierbei setzt man in der Regel soviel des alkalisch wirkenden Mittels ein, die ausreicht, um die 6-Acetoxy-2-naphthoesäure zu lösen. Die alkalische Hydrolyse erfolgt bevorzugt bei einer Temperatur zwischen 50 und 105 °C, insbesondere zwischen 75 und 95 °C, und in einem pH-Bereich zwischen 8 und 14, insbesondere zwischen 11 und 13. Nach der alkalischen Hydrolyse wird die gebildete 6-Hydroxy-2-naphthoesäure — gegebenenfalls nach vorheriger Klärung der Lösung mit üblichen Klärhilfsmitteln, wie Aktivkohle, und anschließender Filtration — mittels einer Säure, wie einer wäßrigen Mineralsäure, so Salzsäure oder bevorzugt Schwefelsäure, ausgefällt ; die Ausfällung erfolgt bei einem pH-Wert zwischen 4 und 1. Die sich ausscheidende 6-Hydroxy-2-naphthoesäure kann in üblicher Weise sodann isoliert werden.

Die Hydrolyse der 6-Acetoxy-2-naphthoesäure in wäßrig-saurem Medium erfolgt in der Regel unter Verwendung einer wäßrigen Mineralsäure, wie Salzsäure und insbesondere Schwefelsäure. Hierbei kann die Konzentration der wäßrigen Säure in weiten Bereichen variieren. Üblicherweise wird eine 1 bis 50 %ige wäßrige Mineralsäure, wie wäßrige Schwefelsäure, als Reaktionsmedium verwendet ; bevorzugt verwendet man eine 10 bis 30 %ige, insbesondere eine 15 bis 20 %ige wäßrige Säure. Der pH des Reaktionsmediums liegt dementsprechend in der Regel unterhalb von 1,5, wie bspw. bei dem Wert 1 oder unterhalb 1. Die Hydrolyse wird in der Regel bei einer Temperatur zwischen 50 und 110 °C, insbesondere zwischen 85 und 105 °C, durchgeführt. Aus dem sauren Hydrolysemedium scheidet sich die gebildete 6-Hydroxy-2-naphthoesäure ab ; sie kann in üblicher Weise isoliert werden.

Die nachfolgenden Beispiele dienen zur näheren Erläuterung der Erfindung. Die Teile sind Gewichtsteile und die Prozentangaben beziehen sich auf Gewichtsprozente, sofern nicht anders vermerkt. Die Bestimmung des Reingehaltes erfolgte durch Hochdruck-Flüssigkeitschromatographie (HPLC)-Analyse.

Beispiel 1

In eine Lösung von 37,6 Teilen (0,2 Mol) 6-Hydroxy-2-naphthoesäure in 750 Teilen einer 2,2 %igen wäßrigen Natronlauge (diese Ausgangslösung besitzt einen pH-Wert von etwa 12,5) werden bei einer Temperatur zwischen 20 und 25 °C 20,4 Teile (0,2 Mol) Essigsäureanhydrid eingerührt. Der Reaktionsansatz wird noch etwa 1 Stunde weitergerührt und anschließend mit verdünnter wäßriger Schwefelsäure auf einen pH-Wert zwischen 5 und 4,5 eingestellt. Das ausgefallene Produkt wird abgesaugt, mit Wasser gewaschen und getrocknet.

Ausbeute an 6-Acetoxy-2-naphthoesäure : 45,2 Teile mit einem Reingehalt von etwa 99 %, entsprechend 98 % d. Th.

Beispiel 2

In eine Lösung von 37,6 Teilen 6-Hydroxy-2-naphthoesäure in 750 Teilen einer 2,2 %igen wäßrigen Natronlauge werden bei einer Temperatur von 20 bis 25 °C 61,2 Teile (0,6 Mol) Essigsäureanhydrid eingerührt. Nach Beendigung der Zugabe wird der Ansatz noch etwa eine Stunde weitergerührt und sodann das bei einem pH-Wert zwischen 5,5 und 4,5 ausgefallene Produkt abgesaugt, mit Wasser gewaschen und getrocknet.

Ausbeute an 6-Acetoxy-2-naphthoesäure : 44,4 Teile mit einem Reingehalt von mindestens 99,5 %, entsprechend 96,5 % d. Th.

Die Beispiele 3 bis 5 demonstrieren die selektive Acetylierung von 6-Hydroxy-2-naphthoesäure im Gemisch aus 6-Hydroxy-2-naphthoesäure und 3-Hydroxy-2-naphthoesäure unterschiedlicher Zusammensetzung :

Beispiel 3

In eine Lösung eines Gemisches aus 90 Teilen (0,478 Mol) 6-Hydroxy-2-naphthoesäure und 10 Teilen 3-Hydroxy-2-naphthoesäure in 2 000 Teilen einer 2,2 %igen wäßrigen Natronlauge (diese Ausgangslösung besitzt einen pH-Wert von etwa 12,5) werden bei 20-25 °C 92 Teile (0,9 Mol) Essigsäureanhydrid eingerührt. Man rührt noch eine Stunde nach und saugt das bei einem pH-Wert zwischen etwa 5,5 und 4,5 ausgefallene Produkt ab, wäscht es mit Wasser nach und trocknet es.

Ausbeute an 6-Acetoxy-2-naphthoesäure : 104,5 Teile mit einem Reingehalt von 99 %, entsprechend 95,5 d. Th.

Das Filtrat kann auf die Nebenprodukte aufgearbeitet werden. Hierzu wird es mit verdünnter wäßriger Schwefelsäure auf einen pH-Wert von 2 gestellt, das ausgefallene Produkt isoliert (beispielsweise durch Filtration) und getrocknet. Man erhält 8,5 Teile eines Produktes das 91 % 3-Hydroxy-2-naphthoesäure enthält ; die « Verunreinigungen » sind hauptsächlich nicht umgesetzte 6-Hydroxy-2-naphthoesäure.

Beispiel 4

Eine Lösung von 50 Teilen 6-Hydroxy-2-naphthoesäure und 50 Teilen 3-Hydroxy-2-naphthoesäure in einem Gemisch aus 2 000 Teilen Wasser und 93 Teilen einer 45 %igen wäßrigen Natronlauge (die Lösung besitzt einen pH-Wert von etwa 12,5) wird unter Rühren bei 20 bis 25 °C mit 50 Teilen Essigsäureanhydrid versetzt. Man rührt noch eine Stunde nach und saugt das bei einem pH-Wert zwischen 6,0 und 5,5 ausgefallene Produkt ab, wäscht es mit Wasser nach und trocknet es.

Ausbeute an 6-Acetoxy-2-naphthoesäure : 57,5 Teile mit einem Reingehalt von etwa 99 %, entsprechend 94 % d. Th.

Die Aufarbeitung des Filtrates erfolgt gemäß Beispiel 3. Man erhält etwa 50 Teile eines Produktes, das etwa 95 % 3-Hydroxy-2-naphthoesäure enthält ; die « Verunreinigungen » sind hauptsächlich nicht umgesetzte 6-Hydroxy-2-naphthoesäure.

Beispiel 5

In eine Lösung mit einem pH-Wert von etwa 12,5 eines Gemisches aus 10 Teilen 6-Hydroxy-2-naphthoesäure und 90 Teilen 3-Hydroxy-2-naphthoesäure in 2 000 Teilen einer 2 %igen wäßrigen Natronlauge werden bei 20 bis 25 °C 45 Teile Essigsäureanhydrid eingerührt. Man rührt den Reaktionsansatz noch eine Stunde nach und saugt das bei einem pH-Wert zwischen etwa 5,5 und 5,0 ausgefallene Produkt ab, wäscht es mit Wasser nach und trocknet es.

Ausbeute an 6-Acetoxy-2-naphthoesäure : 9,1 Teile mit einem Reingehalt von ca. 98 %.

Die Aufarbeitung des Filtrates erfolgt gemäß Beispiel 3. Man erhält etwa 94 Teile eines Produktes, das etwa 95 % 3-Hydroxy-2-naphthoesäure enthält ; die « Verunreinigungen » sind hauptsächlich nicht umgesetzte 6-Hydroxy-2-naphthoesäure.

Beispiel 6

728 Teile wasserfreies 2-Hydroxynaphthalin-kaliumsalz werden nach einem bekannten Carboxylierungsverfahren, beispielsweise bei einer Temperatur zwischen 250 und 270 °C und unter einem Kohlendioxiddruck von 3 bis 4 bar carboxyliert. Die Carboxylierungsschmelze wird mit etwa 7 000 Teilen Wasser ausgelöst und bei 80 °C mit Mineralsäure, wie Salzsäure auf einen pH-Wert von 6,5 gestellt. Die Abtrennung von gebildetem Harz sowie von geringen Mengen an 2-Hydroxynaphthalin erfolgt beispielsweise analog den Angaben der deutschen Offenlegungsschrift 29 11 667.

Anschließend wird die wäßrige Lösung mit wäßriger Natronlauge auf einen pH-Wert von 12,5 gestellt und sodann bei einer Temperatur zwischen 20 und 25 °C mit 270 Teilen Essigsäureanhydrid unter Rühren versetzt und rührt noch etwa 1 Stunde nach, wobei der pH-Wert allmählich auf 6,5 bis 6,0 fällt.

Das ausgefallene Produkt wird isoliert, mit Wasser gewaschen und unter reduziertem Druck getrocknet. Man erhält 330 Teile 6-Acetoxy-2-naphthoesäure mit einem Reingehalt von über 99 %.

Das Filtrat kann auf Nebenprodukte aufgearbeitet werden. Hierzu stellt man es mit verdünnter wäßriger Schwefelsäure auf einen pH-Wert von 2 ein, isoliert das ausgefallene Produkt und trocknet es. Man erhält 60 Teile eines Produktgemisches, das im wesentlichen aus 3-Hydroxy-2-naphthoesäure besteht.

Beispiel 7

100 Teile wasserfeuchte 6-Acetoxy-2-naphthoesäure mit einem Gehalt an 60 Teilen Wasser, wie sie beispielsweise entsprechend dem Beispiel 6 vor der Trocknung erhalten wird, werden in 300 Teilen einer etwa 17 %igen wäßrigen Schwefelsäure vier Stunden zwischen 90 und 100 °C unter Rühren erhitzt. Die sich abscheidende 6-Hydroxy-2-naphthoesäure wird nach Abkühlen des Ansatzes auf 70 bis 80 °C abfiltriert, mit heißem Wasser gewaschen und getrocknet. Man erhält 31 Teile reine 6-Hydroxy-2-naphthoesäure mit einem Schmelzpunkt von 245 °C (umkristallisierte 6-Hydroxy-2-naphthoesäure besitzt gemäß Beispiel 1 der US-PS 1 593 816 einen Schmelzpunkt von 245 °C).

Beispiel 8

Zur Hydrolyse von 6-Acetoxy-2-naphthoesäure verfährt man gemäß den Angaben des Beispieles 7, setzt jedoch anstelle der 17 %igen Schwefelsäure 500 Teile einer etwa 10 %igen wäßrigen Schwefelsäure und gleichzeitig 20 Teile Ethanol ein. Man erhält 28,5 Teile reine 6-Hydroxy-2-naphthoesäure mit einem Schmelzpunkt von 245 °C.

Beispiel 9

100 Teile wasserfeuchte 6-Acetoxy-2-naphthoesäure, wie sie entsprechend dem Beispiel 6 vor der Trocknung erhalten wird, mit einem Gehalt an 60 Teilen Wasser werden in 500 Teilen einer etwa 3,5 %igen wäßrigen Natronlauge zwei Stunden unter Rühren bei einer Temperatur zwischen 80 und 90 °C erwärmt. Anschließend klärt man die erhaltene Lösung durch Behandlung von 5 Teilen Aktivkohle und Filtration, gibt etwa 40 Teile einer etwa 50 %igen wäßrigen Schwefelsäure hinzu und isoliert die abgeschiedene 6-Hydroxy-2-naphthoesäure bei einer Temperatur zwischen 70 und 80 °C, beispielsweise durch Filtration. Das isolierte Produkt wird mit heißem Wasser gewaschen ; nach dem Trocknen erhält man 30 Teile reine 6-Hydroxy-2-naphthoesäure mit einem Schmelzpunkt von 245 °C.

**Patentansprüche**

1. Verfahren zur Herstellung von 6-Acetoxy-2-naphthoesäure, dadurch gekennzeichnet, daß man das Alkalisalz (Dialkalisalz) der 6-Hydroxy-2-naphthoesäure in wäßrig-alkalischer Lösung mit Essigsäureanhydrid umsetzt.

2. Verfahren nach Anspruch 1, dadurch gekennzeichnet, daß die wäßrig-alkalische Lösung der 6-Hydroxy-2-naphthoesäure Beimengungen an einer oder mehreren isomeren Hydroxynaphthoesäuren gelöst enthält.

3. Verfahren nach Anspruch 2, dadurch gekennzeichnet, daß die isomere Hydroxynaphthoesäure oder eine der isomeren Hydroxynaphthoesäuren die 3-Hydroxy-2-naphthoesäure ist.

4. Verfahren zur Abtrennung von 6-Hydroxy-2-naphthoesäure von deren isomeren Hydroxynaphthoesäuren, insbesondere von 3-Hydroxy-2-naphthoesäure, durch Acetylierung der 6-Hydroxy-2-naphthoesäure gemäß Anspruch 1.

5. Verfahren nach einem der Ansprüche 1 bis 4, dadurch gekennzeichnet, daß man die Acetylierung bei einem pH-Wert von 10 oder größer als 10 durchführt.

6. Verfahren nach einem der Ansprüche 1 bis 4, dadurch gekennzeichnet, daß man die Acetylierung bei einem pH-Wert von 10 bis 13 durchführt.

7. Verfahren nach einem der Ansprüche 1 bis 4, dadurch gekennzeichnet, daß man die Acetylierung bei einem pH-Wert zwischen 11,5 und 12,5 durchführt.

8. Verfahren nach einem der Ansprüche 1 bis 7, dadurch gekennzeichnet, daß man die Umsetzung bei einer Temperatur von unterhalb 50 °C durchführt.

9. Verfahren nach einem der Ansprüche 1 bis 7, dadurch gekennzeichnet, daß man die Acetylierung bei einer Temperatur zwischen 15 und 30 °C durchführt.

10. Verfahren nach einem der Ansprüche 1 bis 9, dadurch gekennzeichnet, daß man die Acetylierung unter Verwendung von 1 bis 3,5 Mol Essigsäureanhydrid pro Mol 6-Hydroxy-2-naphthoesäure durchführt.

11. Verfahren nach einem der Ansprüche 1 bis 9, dadurch gekennzeichnet, daß man die Acetylierung unter Verwendung von 1,2 bis 3,0 Mol Essigsäureanhydrid pro Mol 6-Hydroxy-2-naphthoesäure durchführt.

12. Verfahren nach Anspruch 4 zur Abtrennung von 6-Hydroxy-2-naphthoesäure als 6-Acetoxy-2-naphthoesäure von isomeren Hydroxy-naphthoesäuren, insbesondere von 3-Hydroxy-naphthoesäuren, dadurch gekennzeichnet, daß man eine wäßrig-alkalische Lösung der Alkalisalze der 6-Hydroxy-2-naphthoesäure mit den isomeren Hydroxynaphthoesäuren mit Essigsäureanhydrid umsetzt und sodann die gebildete 6-Acetoxy-2-naphthoesäure bei einem pH-Wert zwischen etwa 6,0 und 4,0 ausfällt und isoliert und gegebenenfalls das Filtrat auf einen pH-Wert zwischen 1 und 2 stellt und die ausgefällten isomeren Hydroxynaphthoesäuren, insbesondere 3-Hydroxy-2-naphthoesäure, isoliert.

13. Verfahren nach einem der Ansprüche 1 bis 12, dadurch gekennzeichnet, daß man als wäßrig-alkalische Lösungen von Hydroxy-naphthoesäuren mit der 6-Hydroxy-2-naphthoesäure die aus einem Carboxylierungsansatz von Kalium-β-naphtholat erhältlichen wäßrig-alkalischen Lösungen einsetzt.

14. Verfahren nach einem der Ansprüche 1 bis 13 zur Herstellung von reiner 6-Hydroxy-2-naphthoesäure aus verunreinigter 6-Hydroxy-2-naphthoesäure, dadurch gekennzeichnet, daß man die verunreinigte 6-Hydroxy-2-naphthoesäure zunächst gemäß einer Verfahrensweise einer der Ansprüche 1 und 5 bis 12 acetyliert und die abgetrennte 6-Acetoxy-2-naphthoesäure in wäßrigem Medium hydrolysiert.

15. Verfahren nach Anspruch 14, dadurch gekennzeichnet, daß die Hydrolyse in einer wäßrigen Mineralsäure durchgeführt wird.

16. Verfahren nach Anspruch 14, dadurch gekennzeichnet, daß die Hydrolyse in einer wäßrigen Alkalihydroxidlösung durchgeführt wird.

17. Verfahren nach Anspruch 14, 15 oder 16, dadurch gekennzeichnet, daß die Hydrolyse in Gegenwart eines geradkettigen oder verzweigten Alkanols von 1 bis 5 C-Atomen durchgeführt wird.

**Claims**

1. A process for preparing 6-acetoxy-2-naphthoic acid which comprises reacting the alkali metal salt (di-alkali metal salt) of 6-hydroxy-2-naphthoic acid in aqueous alkaline solution with acetic anhydride.

2. The process as claimed in claim 1, wherein the aqueous alkaline solution of 6-hydroxy-2-naphthoic acid contains admixtures of one or more hydroxynaphthoic acid isomers in dissolved form.

3. The process as claimed in claim 2 wherein the or one of the hydroxynaphthoic acid isomers is 3-hydroxy-2-naphthoic acid.

4. A process for separating 6-hydroxy-2-naphthoic acid from its isomeric hydroxynaphthoic acids, in particular from 3-hydroxy-2-naphthoic acid, by acetylation of 6-hydroxy-2-naphthoic acid according to claim 1.

5. The process as claimed in any one of claims 1 to 4, wherein the acetylation is carried out at a pH of 10 or higher.

6. The process as claimed in any one of claims 1 to 4, wherein the acetylation is carried out at a pH of 10 to 13.

7. The process as claimed in any one of claims 1 to 4, wherein the acetylation is carried out at a pH between 11.5 and 12.5.

8. The process as claimed in any one of claims 1 to 7, wherein the acetylation is carried out at a temperature of below 50 °C.

9. The process as claimed in any one of claims 1 to 7, wherein the acetylation is carried out at a temperature between 15 and 30 °C.

10. The process as claimed in any one of claims 1 to 9, wherein the acetylation is carried out using 1 to 3.5 moles of acetic anhydride per mole of 6-hydroxy-2-naphthoic acid.

11. The process as claimed in any one of claims 1 to 9, wherein the acetylation is carried out using 1.2 to 3.0 moles of acetic anhydride per mole of 6-hydroxy-2-naphthoic acid.

12. The process as claimed in claim 4 for separating 6-hydroxy-2-naphthoic acid in the form of 6-acetoxy-2-naphthoic acid from isomeric hydroxynaphthoic acids, in particular from 3-hydroxynaphthoic acids, wherein an aqueous alkaline solution of alkali metal salts of 6-hydroxy-2-naphthoic acid which contains the isomeric hydroxynaphthoic acids is reacted with acetic anhydride and the resulting 6-acetoxy-2-naphthoic acid is then precipitated at a pH between about 6.0 and 4.0 and is isolated, and if desired the filtrate is brought to a pH between 1 and 2 and the precipitated isomeric hydroxynaphthoic acids, in particular 3-hydroxy-2-naphthoic acid, are isolated.

13. The process as claimed in any one of claims 1 to 12, wherein the aqueous alkaline solutions of hydroxynaphthoic acids, including 6-hydroxy-2-naphthoic acid, are the aqueous alkaline solutions obtainable from a carboxylation of potassium β-naphtholate.

14. A process according to one of claims 1 to 13 for the preparation of pure 6-hydroxy-2-naphthoic acid from impure 6-hydroxy-2-naphthoic acid, which comprises firstly acetylating the soiled 6-hydroxy-2-naphthoic acid according to a procedure of one of claims 1 and 5 to 12, isolating the 6-acetoxy-2-naphthoic acid formed, and hydrolyzing it in an aqueous medium.

15. A process according to claim 14 wherein the hydrolysis reaction is carried out in an aqueous mineral acid.

16. A process according to claim 14 wherein the hydrolysis reaction is carried out in an aqueous alkali metal hydroxide solution.

17. A process according to claim 14, 15 or 16 wherein the hydrolysis reaction is carried out in the presence of a straight-chained or branched alkanol of from 1 to 5 carbon atoms.

## Revendications

1. Procédé pour préparer l'acide acétoxy-6 naphtalène-carboxylique-2, procédé caractérisé en ce qu'on fait réagir un sel de métal alcalin (disel de métal alcalin) de l'acide hydroxy-6 naphtalène-carboxylique-2, en solution aqueuse alcaline, avec l'anhydride acétique.

2. Procédé selon la revendication 1 caractérisé en ce que la solution aqueuse alcaline de l'acide hydroxy-6 naphtalène-carboxylique-2 contient, en solution, des impuretés constituées d'un ou de plusieurs acides hydroxy-naphtoïques isomères.

3. Procédé selon la revendication 2 caractérisé en ce que l'acide hydroxy-naphtoïque isomère ou l'un des acides hydroxy-naphtoïques isomères est l'acide hydroxy-3 naphtalène-carboxylique-2.

4. Procédé pour séparer l'acide hydroxy-6 naphtalène-carboxylique-2 des acides hydroxy-naphtoïques qui lui sont isomères, plus particulièrement de l'acide hydroxy-3 naphtalène-carboxylique-2, par acétylation de l'acide hydroxy-6 naphtalène-carboxylique-2 selon la revendication 1.

5. Procédé selon l'une quelconque des revendications 1 à 4, caractérisé en ce que l'acétylation est effectuée à un pH au moins égal à 10.

6. Procédé selon l'une quelconque des revendications 1 à 4, caractérisé en ce que l'acétylation est effectuée à un pH de 10 à 13.

7. Procédé selon l'une quelconque des revendications 1 à 4, caractérisé en ce que l'acétylation est effectuée à un pH compris entre 11,5 et 12,5.

8. Procédé selon l'une quelconque des revendications 1 à 7, caractérisé en ce que la réaction est effectuée à une température inférieure à 50 °C.

9. Procédé selon l'une quelconque des revendications 1 à 7, caractérisé en ce que l'acétylation est effectuée à une température comprise entre 15 et 30 °C.

10. Procédé selon l'une quelconque des revendications 1 à 9, caractérisé en ce qu'on effectue l'acétylation en utilisant de 1 à 3,5 mol d'anhydride acétique par mole de l'acide hydroxy-6 naphtalène-carboxylique-2.

11. Procédé selon l'une quelconque des revendications 1 à 9, caractérisé en ce qu'on effectue l'acétylation en utilisant de 1,2 à 3,0 mol d'anhydride acétique par mole de l'acide hydroxy-6 naphtalène-carboxylique-2.

12. Procédé selon la revendication 4 pour séparer l'acide hydroxy-6 naphtalène-carboxylique-2, sous la forme de l'acide acétoxy-6 naphtalène-carboxylique-2, d'acides hydroxy-naphtoïques isomères, plus particulièrement d'acides hydroxy-3 naphtoïques, procédé caractérisé en ce qu'on fait réagir avec l'anhydride acétique une solution aqueuse alcaline de sels de métaux alcalins de l'acide hydroxy-6 naphtalène-carboxylique-2 accompagné des acides hydroxy-naphtoïques isomères, puis on fait précipiter, à un pH compris entre environ 6,0 et 4,0, l'acide acétoxy-6 naphtalène-carboxylique-2 formé, on l'isole et éventuellement on porte le pH du filtrat à une valeur comprise entre 1 et 2 et on isole les acides hydroxy-naphtoïques isomères qui ont précipité, plus particulièrement l'acide hydroxy-3 naphtalène-carboxylique-2.

13. Procédé selon l'une quelconque des revendications 1 à 12, caractérisé en ce qu'on utilise, comme solutions aqueuses alcalines d'acides hydroxy-naphtoïques comprenant l'acide hydroxy-6 naphtalène-carboxylique-2, les solutions aqueuses alcalines qui peuvent être obtenues dans une opération de carboxylation du β-naphtolate de potassium.

14. Procédé selon l'une quelconque des revendications 1 à 13 pour préparer l'acide hydroxy-6 naphtalène-carboxylique-2 pur à partir d'un acide hydroxy-6 naphtalène-carboxylique-2 souillé, procédé caractérisé en ce qu'on acétyle d'abord, en opérant selon l'une des revendications 1 et 5 à 12, l'acide hydroxy-6 naphtalène-carboxylique-2 souillé, et on hydrolyse en milieu aqueux l'acide acétoxy-6 naphtalène-carboxylique-2 séparé.

15. Procédé selon la revendication 14 caractérisé en ce qu'on effectue l'hydrolyse dans un acide minéral aqueux.

16. Procédé selon la revendication 14 caractérisé en ce qu'on effectue l'hydrolyse dans une solution aqueuse d'un hydroxyde de métal alcalin.

17. Procédé selon l'une quelconque des revendications 14, 15 et 16, caractérisé en ce qu'on effectue l'hydrolyse en présence d'un alcanol linéaire ou ramifié qui contient de 1 à 5 atomes de carbone.